# EUROPÄISCHE PATENTSCHRIFT

(11) **EP 0 556 156 B1**
(45) Veröffentlichungstag und Bekanntmachung des Hinweises auf die Patenterteilung: **25.09.1996**
(21) Anmeldenummer: 93810070.8
(22) Anmeldetag: 04.02.1993
(51) Int. Cl.: C07D 251/22

(54) **Verfahren zur Herstellung von 2-Hydroxy-4,6-diaryl-1,3,5-triazin**
Process for the preparation of 2-hydroxy-4,6-diaryl-1,3,5-triazine
Procédé pour la préparation de 2-hydroxy-4,6-diaryl-1,3,5-triazine

(30) Priorität: 13.02.1992 CH 426/92
(43) Veröffentlichungstag der Anmeldung: 18.08.1993
(73) Patentinhaber: CIBA-GEIGY AG, 4002 Basel (CH)
(72) Erfinder: Reinehr, Dieter, Dr., W-7842 Kandern (DE); Bacher, Jean-Pierre, F-68220 Buschwiller (FR)

(56) Entgegenhaltungen:
- DE-B- 1 193 057
- BERICHTE DER DEUTSCHEN CHEMISCHEN GESELLSCHAFT, Bd. 23, Nr. 13, 1890, BERLIN Seiten 2919 - 2922 A. PINNER 'Ueber Diphenyloxykyanidin'
- JOURNAL OF THE CHEMICAL SOCIETY D, CHEMICAL COMMUNICATIONS, Nr. 10, 1971, Seiten 498 - 499 B. SINGH ET AL. 'Simple New Synthesis of 4,6-Diaryl-2-hydroxy-s-triazines and Amidines'
- JOURNAL OF HETEROCYCLIC CHEMISTRY, Bd. 13, Nr. 4, 1976, Seiten 917 - 919 D. ALSOFROM ET AL. 'A New Synthesis of s-Triazines'
- JOURNAL OF ORGANIC CHEMISTRY, Bd. 42, Nr. 14, 1977, Seiten 2530 - 2532 E.P. PAPADOPOULOS ET AL. 'Heterocycles from N-Ethoxycarbonylthioamides and Dinucleophilic Reagents. 3. Six- and Seven-Membered Rings with Two or Three Heteroatoms'

## Beschreibung

Die vorliegende Erfindung betrifft ein Ein-Topf-Verfahren zur Herstellung von 2-Hydroxy-4,6-diaryl-1,3,5-triazin ausgehend von einfachen Ausgangsverbindungen wie substituierten Benzonitrilen und Halogenkohlensäurealkylestern und Natriumamid.

Es ist bereits aus J. Chem. Soc. D, Chem. Commun. 1971 (10), Seiten 498-499 oder J. Heterocycl. Chem. 13 (4), Seiten 917-919 (1976) bekannt, 2-Hydroxy-4,6-diaryl-1,3,5-triazine durch Umsetzung aromatischer Nitrile mit Harnstoff oder Harnstoffabkömmlingen wie Guanidin, Biguanid, Biuret oder Thioharnstoff herzustellen. Diese Synthese vermag jedoch nicht voll zu überzeugen, sodass Bedarf nach neuen verbesserten Herstellungsverfahren für 2-Hydroxy-4,6-diaryl-1,3,5-triazine besteht.

Gegenstand der vorliegenden Erfindung ist daher ein Verfahren zur Herstellung eines 2-Hydroxy-4,6-diaryl-1,3,5-triazins der Formel 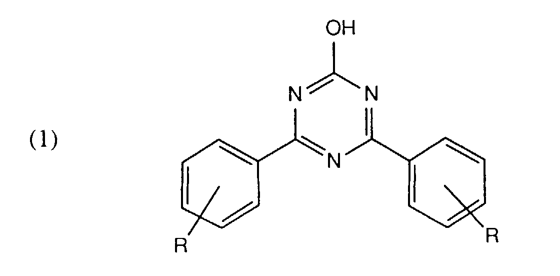 ausgehend von einer aromatischen Nitrilverbindung, das dadurch gekennzeichnet ist, dass man jeweils äquimolare Mengen der aromatischen Nitrilverbindung mit einem Halogenameisensäurealkylester und einem Alkalimetallamid nach folgendem Schema 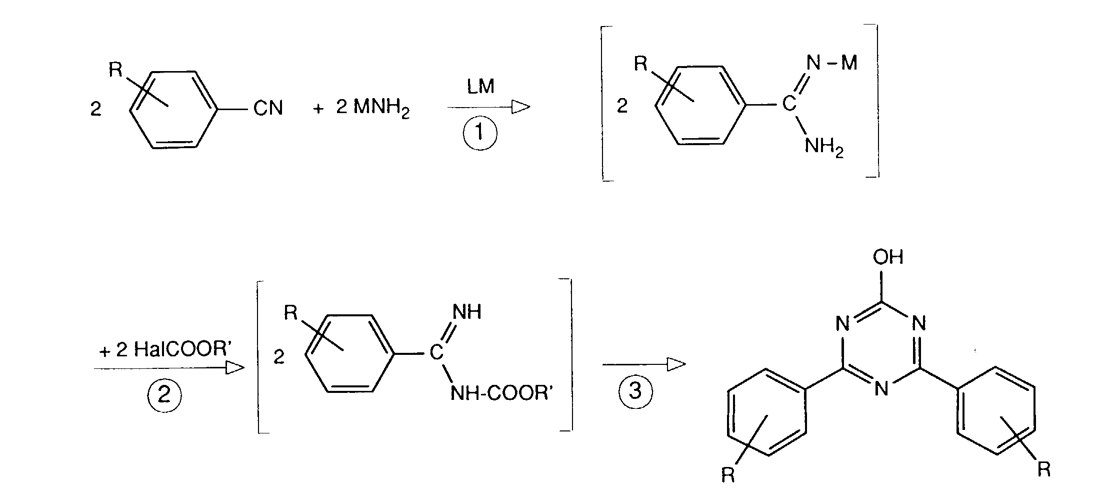 in einem Ein-Topf-Verfahren ohne Isolierung der Zwischenprodukte umsetzt, wobei die Reaktion der ersten Stufe bei einer Temperatur zwischen 50 und 150°C, die Reaktion der zweiten Stufe bei einer Temperatur zwischen 5 und 50°C, und die Reaktion der dritten Stufe in einem Bereich von 120 bis 230°C durchgeführt wird, und worin R Wasserstoff, Halogen, C₁-C₄-Alkyl, oder C₁-C₄Alkoxy, R' C₁-C₄Alkyl, Hal ein Halogenatom, M Alkalimetall und LM ein inertes, organisches Lösungsmittel mit einem Siedepunkt von 160 bis 250°C bedeuten.

C₁-C₄Alkyl steht beispielsweise für Methyl, Ethyl, n-Propyl, Isopropyl, n-Butyl, Isobutyl, sek.Butyl oder tert.Butyl, C₁-C₄Alkoxy für Methoxy, Ethoxy, Isopropoxy, Isobutoxy oder tert.Butoxy.

Halogen bedeutet Fluor, Chlor oder Brom, wobei Chlor bevorzugt ist.

Als Basen können für das erfindungsgemässe Verfahren Lithium-, Kalium- oder Natriumamid verwendet werden, wobei Natriumamid in Form einer 60-70%igen Suspension bevorzugt eingesetzt wird.

Als Beispiele für die als Ausgangsverbindungen eingesetzten aromatischen Nitrile seien Benzonitril, Methylbenzonitril, Ethylbenzonitril, Propylbenzonitril, n-Butylbenzonitril, tert.Butylbenzonitril und Chlorbenzonitril zu nennen. Bevorzugte Ausgangsverbindungen sind Benzonitril, 3-Methylbenzonitril, 4-Methylbenzonitril, 3-Chlorbenzonitril oder 4-Chlorbenzonitril, wobei Benzonitril im Vordergrund des Interesses steht.

Als Beispiele für Halogenameisensäurealkylester sind Bromameisensäuremethyl-, Chlorameisensäuremethyl-, Bromameisensäureethyl- oder Chlorameisensäureethylester zu nennen. Die letztgenannte Verbindung wird in dem erfindungsgemässen Verfahren bevorzugt eingesetzt.

Als inerte, organischen Lösungsmittel kommen beispielsweise in Betracht: o-Dichlorbenzol, Nitrobenzol, 1,2,3-Trimethylbenzol, ein Gemisch aus 26,5 Gew.% Diphenyl und 73,5 Gew.% Diphenylether (®Dowtherm) oder Anisol. Als bevorzugt verwendetes Lösungsmittel ist ®Dowtherm zu nennen.

Die Reaktion der ersten Stufe wird bei einer Temperatur zwischen 50 und 150°C, vorzugsweise 70 bis 120°C, die Reaktion der zweiten Stufe bei einer Temperatur zwischen 5 und 50, vorzugsweise 10 und 40°C, und die Reaktion der dritten Stufe in einem Bereich von 120 bis 230, vorzugsweise 50 bis 200°C durchgeführt.

Der Ringschluss zum 2-Hydroxy-4,6-diaryl-1,3,5-triazin wird zwecks rascher Entfernung des Ethanols und Urethans auch vorteilhafterweise unter vermindertem Druck zwischen 150 und 200 mbar durchgeführt.

Die Verbindungen entsprechend Formel (1) sind bekannt, z.B. aus A. Pinner, Chem. Ber. 23, 2919 (1890).

Gegenüber dem dort beschriebenen Verfahren werden die Verbindungen der Formel (1) nach dem erfindungsgemässen Verfahren ohne Isolierung des Benzimidoyl-carbaminsäurealkylesters in einem einstufigen Verfahren erhalten. Außerdem wird das Hydroxydiphenyl-1,3,5-triazin entsprechend Formel (1) in so guter Reinheit erhalten, dass es mit Vorteil ohne weitere Isolierung weiterverarbeitet werden kann.

Die nach dem erfindungsgemässen Verfahren hergestellten Triazinverbindungen dienen als Zwischenprodukt für die Herstellung von UV-Absorbern.

In den folgenden Beispielen wird die Erfindung veranschaulicht, ohne sie auf diese zu beschränken.

### Beispiel 1: Herstellung von 2-Hydroxy-4,6-diphenyl-1,3,5-triazin

100 g (2,4 Mol) Natriumamid (95%ig) werden während 12 Stunden in 500 ml ®Dowtherm (Gemisch aus 26,5 Gew.% Diphenyl und 73,5 Gew.% Diphenylether) bei Raumtemperatur in Anwesenheit von Glasperlen gerührt. Die Glasperlen werden sodann abgetrennt und mit weiteren 500 ml ®Dowtherm gewaschen. Die gebildete Suspension wird auf 90°C erwärmt und innerhalb von 1 Stunde 232 g (2,35 Mol) Benzonitril so zugetropft, dass die Temperatur nicht über 105°C ansteigt. Es wird noch 3 Stunden bei 95°C gerührt, dann auf 15°C abgekühlt und innerhalb von 2 Stunden 260,4 g (2,4 Mol) Chlorameisensäureethylester zugetropft. Es wird dann auf 170°C erwärmt und der Druck auf 150 bis 80 mbar abgesenkt, wobei 300 g Destillat bestehend aus Ethanol, Urethan und ®Dowtherm überdestilliert. Nach einer Reaktionszeit von 2 Stunden wird abgekühlt, abgenutscht und mit 1 Liter Methanol gewaschen. Man erhält 255,4 g eines weißen Produktes der Formel 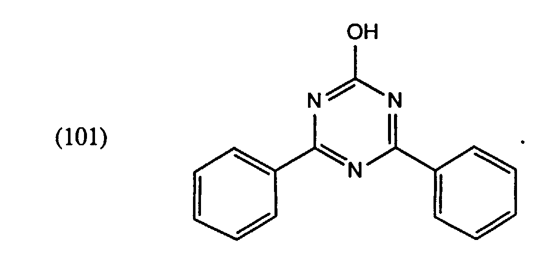 Ausbeute: 90,2 % d. Th.
Fp. 296-297°C

Beispiel 2 bis 5: Man verfährt wie in Beispiel 1 angegeben, mit dem Unterschied, dass man anstelle von 2,35 Mol Benzonitril jeweils 2,35 Mol der in Tabelle I aufgeführten substituierten Benzonitrile einsetzt:

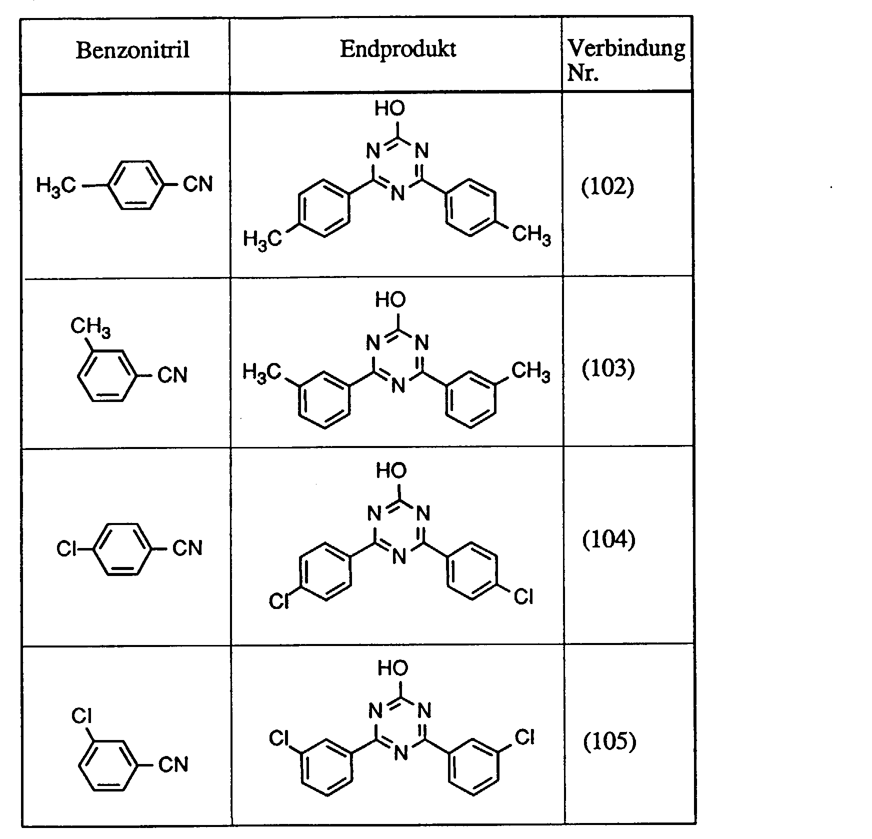

Man erhält die 2-Hydroxy-4,6-diaryl-1,3,5-triazine der Formeln (102) bis (105) in vergleichbaren Ausbeuten.

## Patentansprüche

1. Verfahren zur Herstellung eines 2-Hydroxy-4,6-diaryl-1,3,5-triazins der Formel 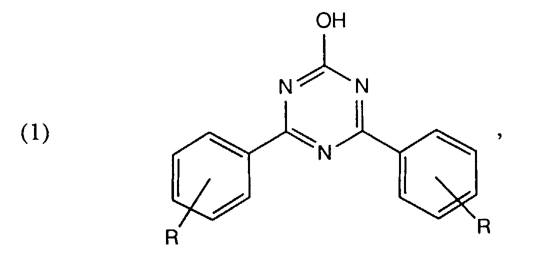 ausgehend von einer aromatischen Nitrilverbindung, dadurch gekennzeichnet, dass man jeweils äquimolare Mengen der aromatischen Nitrilverbindung mit einem Halogenameisensäurealkylester und einem Alkalimetallamid nach folgendem Schema 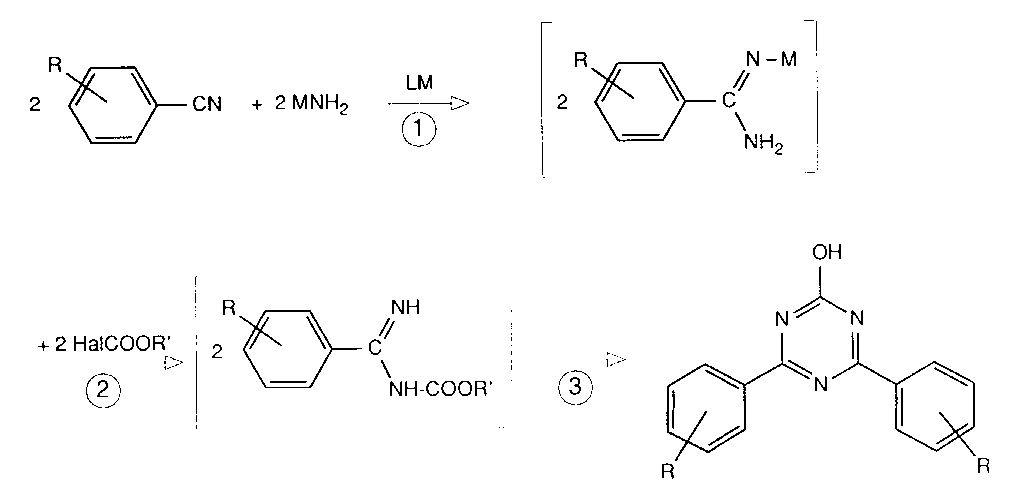 in einem Ein-Topf-Verfahren ohne Isolierung der Zwischenprodukte umsetzt, wobei die Reaktion der ersten Stufe bei einer Temperatur zwischen 50 und 150°C, die Reaktion der zweiten Stufe bei einer Temperatur zwischen 5 und 50°C, und die Reaktion der dritten Stufe in einem Bereich von 120 bis 230°C durchgeführt wird, und worin
R Wasserstoff, Halogen, C₁-C₄Alkyl, oder C₁-C₄Alkoxy, R' C₁-C₄Alkyl, Hal ein Halogenatom, M Alkalimetall und LM ein inertes, organisches Lösungsmittel mit einem Siedepunkt von 160 bis 250°C bedeuten.

2. Verfahren gemäss Anspruch 1, dadurch gekennzeichnet dass man als Alkalimetallamid Natriumamid verwendet.

3. Verfahren gemäss einem der Ansprüche 1 oder 2, dadurch gekennzeichnet, dass man als aromatisches Nitril Benzonitril, 3-Methylbenzonitril 4-Methylbenzonitril, 3-Chlorbenzonitril oder 4-Chlorbenzonitril verwendet.

4. Verfahren gemäss Anspruch 3, dadurch gekennzeichnet dass man als aromatisches Nitril Benzonitril verwendet.

5. Verfahren gemäss einem der Ansprüche 1 bis 4, dadurch gekennzeichnet, dass man als Halogenameisensäurealkylester Chlorameisensäurealkylester verwendet.

6. Verfahren gemäss einem der Ansprüche 1 bis 5, dadurch gekennzeichnet, dass man als inertes organisches Lösungsmittel o-Dichlorbenzol, Nitrobenzol, 1,2,3-Trimethylbenzol, ein Gemisch aus 26,5 Gew.% Diphenyl und 73,5 Gew.% Diphenylether (®Dowtherm) oder Anisol verwendet.

7. Verfahren gemäss Anspruch 6, dadurch gekennzeichnet, dass man als Lösungsmittel ein Gemisch aus 26,5 Gew.% Diphenyl und 73,5 Gew.% Diphenylether (®Dowtherm) verwendet.

8. Verfahren gemäss einem der Ansprüche 1 bis 7, dadurch gekennzeichnet, dass die Reaktion der ersten Stufe bei einer Temperatur zwischen 50 und 150°C, die Reaktion der zweiten Stufe bei einer Temperatur zwischen 5 und 50°C und die Reaktion der dritten Stufe bei einer Temperatur zwischen 120 und 230°C durchgeführt wird.

9. Verfahren gemäss einem der Ansprüche 1 bis 8, dadurch gekennzeichnet, dass die Ringschlussreaktion entsprechend Stufe 3 unter vermindertem Druck zwischen 50 und 200 mbar durchgeführt wird.

10. Verwendung der gemäss Anspruch 1 hergestellten 2-Hydroxy-4,6-diaryl-1,3,5-Triazinverbindung als Zwischenprodukt für die Herstellung von UV-Absorbern.

## Claims

1. A process for the preparation of a 2-hydroxy-4,6-diaryl-1,3,5-triazine of formula 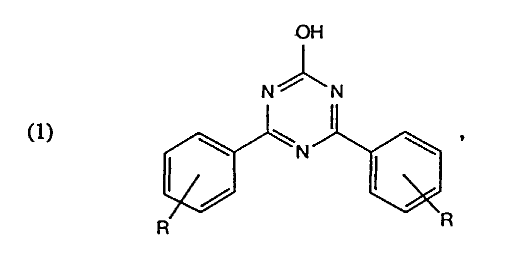 starting from an aromatic nitrile compound, which comprises reacting the aromatic nitrile compound with an alkyl haloformate and an alkali metal amide, using an equimolar amount of each, in accordance with the following scheme 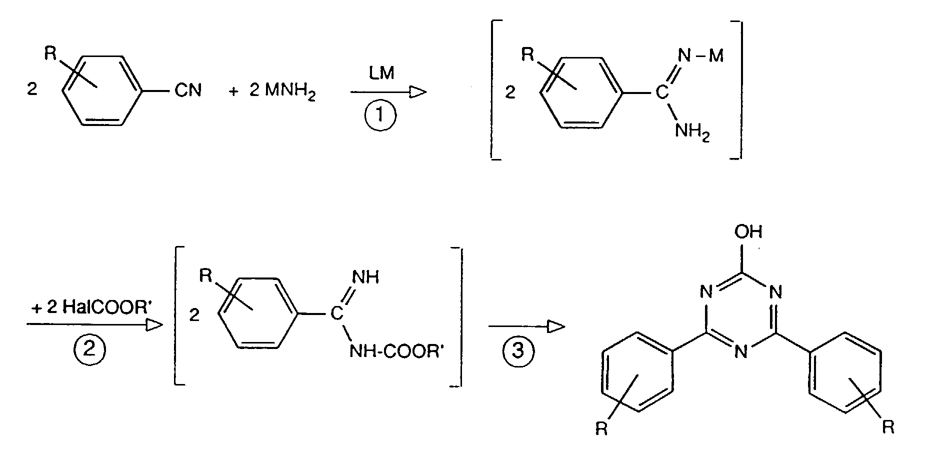 in a one-pot process without isolating the intermediates, where the reaction of the first step is carried out in the temperature range from 50 to 150°C, the reaction of the second step in the temperature range from 5 to 50°C, and the reaction of the third step in a range from 120 to 230°C, and wherein
R is hydrogen, halogen, C₁-C₄alkyl or C₁-C₄alkoxy, R' is C₁-C₄alkyl, Hal is a halogen atom, M is an alkali metal, and LM is an inert organic solvent having a boiling point of from 160 to 250°C.

2. A process according to claim 1, wherein the alkali metal amide is sodium amide.

3. A process according to either claim 1 or claim 2, wherein the aromatic nitrile is benzonitrile, 3-methylbenzonitrile, 4-methylbenzonitrile, 3-chlorobenzonitrile or 4-chlorobenzonitrile.

4. A process according to claim 3, wherein the aromatic nitrile is benzonitrile.

5. A process according to any one of claims 1 to 4, wherein the alkyl haloformate is an alkyl chloroformate.

6. A process according to any one of claims 1 to 5, wherein the inert organic solvent is o-dichlorobenzene, nitrobenzene, 1,2,3-trimethylbenzene, a mixture of 26.5% by weight of diphenyl and 73.5% by weight of diphenyl ether (®Dowtherm) or anisole.

7. A process according to claim 6, wherein the solvent is a mixture of 26.5% by weight of diphenyl and 73.5% by weight of diphenyl ether (®Dowtherm).

8. A process according to any one of claims 1 to 7, wherein the reaction of the first step is carried out in the temperature range from 50 to 150°C, the reaction of the second step in the temperature range from 5 to 50°C, and the reaction of the third step in the temperature range from 120 to 230°C.

9. A process according to any one of claims 1 to 8, wherein the cyclisation of step 3 is carried out under reduced pressure in the range from 50 to 200 mbar.

10. Use of a 2-hydroxy-4,6-diaryl-1,3,5-triazine compound prepared according to claim 1 as intermediate for the synthesis of UV absorbers.

## Revendications

1. Procédé de préparation d'une 2-hydroxy-4,6-diaryl-1,3,5-triazine de formule 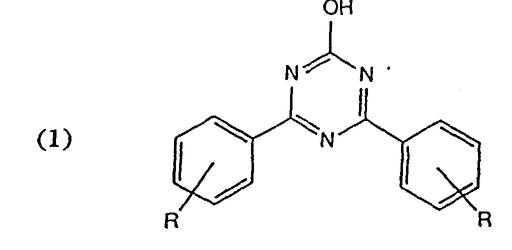 partant d'un composé de type nitrile aromatique, caractérisé en ce que l'on fait réagir des quantités équimolaires du composé de type nitrile aromatique et d'un halogénoformiate d'alkyle et d'un amidure de métal alcalin selon le schéma suivant 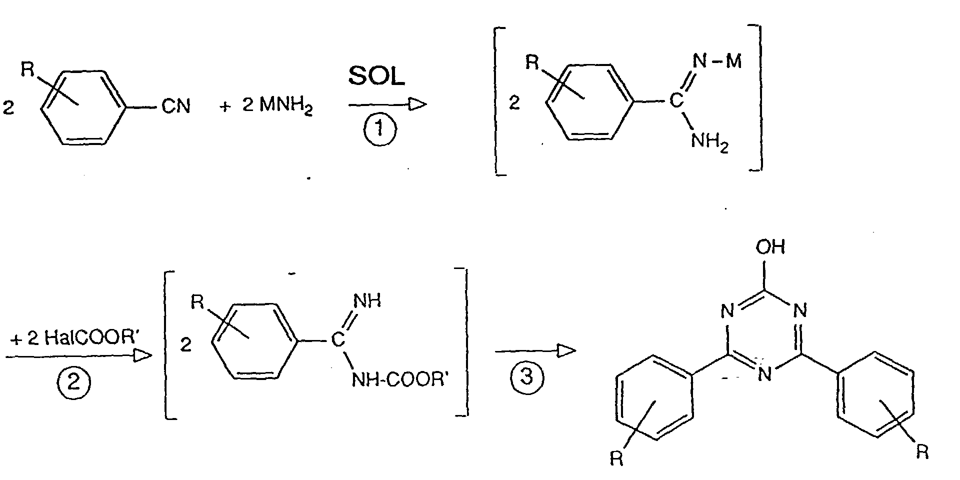 selon un procédé à récipient unique sans isolement des produits intermédiaires, la réaction de la première étape étant effectuée à une température comprise entre 50 et 150 °C, la réaction de la deuxième étape étant effectuée à une température comprise entre 5 et 50 °C et la réaction de la troisième étape étant effectuée à une température comprise entre 120 et 230 °C, et dans laquelle R représente un atome d'hydrogène ou d'halogène, un groupe alkyle en C₁₋₄ ou alcoxy en C₁₋₄, R' représente un groupe alkyle en C₁₋₄, Hal un atome d'halogène, M un métal alcalin et SOL un solvant organique inerte ayant un point d'ébullition compris entre 160 et 250 °C.

2. Procédé conforme à la revendication 1, caractérisé en ce que l'on utilise, comme amidure de métal alcalin, de l'amidure de sodium.

3. Procédé conforme à une des revendications 1 ou 2, caractérisé en ce que l'on utilise, comme nitrile aromatique, du benzonitrile, du 3-méthylbenzonitrile, du 4-méthylbenzonitrile, du 3-chorobenzonitrile ou du 4-chlorobenzonitrile.

4. Procédé conforme à la revendication 3, caractérisé en ce que l'on utilise comme nitrile aromatique du benzonitrile.

5. Procédé conforme à une des revendications 1 à 4, caractérisé en ce que l'on utilise comme halogénoformiate d'alkyle, du chloroformiate d'alkyle.

6. Procédé conforme à une des revendications 1 à 5, caractérisé en ce que l'on utilise comme solvant organique inerte, l'o-dichlorobenzène, le nitrobenzène, le 1,2,3-triméthylbenzène, un mélange contenant 26,5 % en poids de diphényle et de 73,5 % en poids de diphényléther (Dowtherm®) ou l'anisol.

7. Procédé conforme à la revendication 6, caractérisé en ce que l'on utilise comme solvant un mélange de 26,5 % en poids de diphényle et 73,5 % en poids de diphényléther (Dowtherm®).

8. Procédé conforme à une des revendications 1 à 7, caractérisé en ce que la réaction de la première étape est effectuée à une température comprise entre 50 et 150 °C, la réaction de la deuxième étape est réalisée à une température comprise entre 5 et 50 °C et la réaction de la troisième étape dans un domaine de température allant de 120 à 230 °C.

9. Procédé conforme à une des revendications 1 à 8, caractérisé en ce que la réaction de fermeture de cycle correspondant à l'étape 3 est effectuée sous pression réduite comprise entre 50 et 200 mbar.

10. Utilisation du composé de type 2-hydroxy-4,6-diaryl-1,3,5-triazine comme produit intermédiaire pour la préparation d'agents absorbant le rayonnement UV.
